# EUROPEAN PATENT APPLICATION

(11) **EP 4 273 227 A1**
(43) Date of publication of application: **08.11.2023**
(21) Application number: 21914493.8
(22) Date of filing: 29.12.2021
(51) Int. Cl.: C12N 1/21, C12N 15/31, C07K 14/34, C12P 13/14, C12R 1/15

(54) **RECOMBINANT STRAIN FOR PRODUCING L-GLUTAMIC ACID BY MEANS OF MODIFYING GENE BBD29_04920, AND CONSTRUCTION METHOD AND USE THEREOF**

(30) Priority: 30.12.2020 CN 202011631254
(71) Applicant: Inner Mongolia Eppen Biotech Co., Ltd., Chifeng, Inner Mongolia 024000 (CN)
(72) Inventor: MENG, Gang, Chifeng, Inner Mongolia 024000 (CN); JIA, Huiping, Chifeng, Inner Mongolia 024000 (CN); WEI, Aiying, Chifeng, Inner Mongolia 024000 (CN); ZHAO, Chunguang, Chifeng, Inner Mongolia 024000 (CN); SU, Houbo, Chifeng, Inner Mongolia 024000 (CN); YANG, Lipeng, Chifeng, Inner Mongolia 024000 (CN); MA, Fengyong, Chifeng, Inner Mongolia 024000 (CN); ZHOU, Xiaoqun, Chifeng, Inner Mongolia 024000 (CN); GUO, Xiaowei, Chifeng, Inner Mongolia 024000 (CN); TIAN, Bin, Chifeng, Inner Mongolia 024000 (CN)
(74) Representative: ABG Intellectual Property Law, S.L.
(86) International application number: PCT/CN2021/142438
(87) International publication number: WO 2022/143761

(57) **Abstract**

Provided is a method for introducing a point mutation to a BBD29_04920 gene coding sequence in *Corynebacterium* or improving the expression thereof. The point mutation causes a mutation to the base at position 1560 in the BBD29_04920 gene sequence from cytosine (C) to adenine (A) such that asparagine at position 520 of a coded corresponding amino acid sequence is substituted by lysine. The method can increase fermentation yield of glutamic acid in a strain with the mutation. Also provided are the bacterium generating L-glutamic acid, a nucleic acid and protein comprising the mutation, a recombinant vector and recombinant strain comprising the nucleic acid, and use of these biomaterials in the regulation of the production of L-glutamic acid of a microorganism.

## Description

### Technical Field

The present invention belongs to the technical field of gene engineering and microorganisms, and in particular relates to recombinant strain for producing L-glutamic acid, and method for constructing the same and use thereof.

### Background Art

L-Glutamic acid is an important amino acid, which is used in food, clinical medicine and other aspects. Traditionally, L-glutamic acid is mainly produced by fermentation with L-glutamic acid-producing bacteria belonging to the genus *Brevibacterium, Corynebacterium* or *Microtatobiotes,* or the mutants thereof. L-Glutamic acid is generated by biosynthesis with α-ketoglutaric acid, an intermediate product of citric acid cycle in microbial cells. There are two biosynthetic pathways to form L-glutamic acid from α-ketoglutaric acid through the assimilation of ammonium ions. One pathway is to synthesize L-glutamic acid by catalysis with glutamate dehydrogenase (GDH) in the presence of ammonium ions at a high concentration. The other pathway (GS/GOGAT pathway) is to synthesize L-glutamic acid by glutamine synthetase and glutamine-oxoglutaric acid amino transferase. Glutamine synthetase (GS) catalyzes the conversion of L-glutamic acid and ammonium ions to glutamine; glutamine-oxoglutaric acid amino transferase, also known as "glutamate synthetase" (GOGAT), catalyzes the synthesis of L-glutamic acid, in which two molecules of L-glutamic acid are synthesized from one molecule of glutamine which has been synthesized by GS and one molecule of α-ketoglutaric acid.

The improvement in L-amino acid production by fermentation may relate to fermentation techniques such as stirring and supplying oxygen; or to the composition of the nutrient medium, such as the sugar concentration during fermentation; or to processing the fermentation broth into a product in a suitable form, for example, by drying and pelletizing the fermentation broth or ion exchange chromatography; or may relate to the intrinsic properties of the relevant microorganisms themselves in performance.

Methods for improving the properties of these microorganisms in performance include mutagenesis, mutant selection and screening. The strain obtained in this way is resistant to antimetabolites or auxotrophic for metabolites with regulatory importance and produces L-amino acids, and the like.

Although there are a significant amount of methods capable of enhancing the production capacity of L-glutamic acid, it remains necessary to develop methods to produce L-glutamic acid in order to meet the increasing demand.

### Summary

The objective of the present invention is to develop a new technique for enhancing L-glutamic acid production capacity of a bacterium, thereby providing a method for producing L-glutamic acid effectively.

In order to achieve the above objective, the inventors of the present invention have found in research that the gene BBD29_04920 or a homologous gene thereof in a bacterium can be modified, or improved with respect to the expression thereof to enable an enhanced capacity in L-glutamic acid production. The present invention is completed in view of these discoveries.

The present invention provides a bacterium for generating L-glutamic acid, wherein the expression of a polynucleotide encoding an amino acid sequence of SEQ ID NO: 3 or a homologous sequence thereof is improved. The present invention further provides a method for producing L-glutamic acid with the microorganism.

A first aspect of the present invention provides a bacterium for generating L-glutamic acid, having an improved expression of a polynucleotide encoding an amino acid sequence of SEQ ID NO: 3 or a homologous sequence thereof. According to the present invention, the improved expression is an enhanced expression of the polynucleotide, or having a point mutation in the polynucleotide encoding an amino acid sequence of SEQ ID NO: 3 or a homologous sequence thereof, or having a point mutation in, and an enhanced expression of the polynucleotide encoding an amino acid sequence of SEQ ID NO: 3 or a homologous sequence thereof.

The amino acid sequence of SEQ ID NO: 3 or the homologous sequence thereof is a protein coded by the gene BBD29_04920 or a homologous gene thereof.

The bacterium has an enhanced L-glutamic acid production capacity compared with an unmodified strain.

In the present invention, the term "bacterium having L-glutamic acid production capacity" refers to a bacterium having such a capacity of producing and accumulating target L-glutamic acid in a culture medium and/or in a cell of the bacterium that L-glutamic acid can be collected when the bacterium is cultured in the culture medium. A bacterium having L-glutamic acid production capacity can be a bacterium capable of accumulating target L-glutamic acid in a culture medium and/or in a cell of the bacterium in an amount greater than that with an unmodified strain.

The term "unmodified strain" refers to a control strain which has not been modified in a manner such that a specific feature is incorporated. That is, examples of the unmodified strain comprise a wild-type strain and a parent strain.

A bacterium having L-glutamic acid production capacity can be a bacterium capable of accumulating target L-glutamic acid in a culture medium in an amount, preferably above 0.5 g/L, and more preferably above 1.0 g/L.

In the present invention, the term "L-glutamic acid" refers to L-glutamic acid in a free form, a salt thereof or a mixture thereof, unless otherwise specified.

The polynucleotide can encode an amino acid sequence having about 90% or more, about 92% or more, about 95% or more, about 97% or more, about 98% or more, or about 99% or more sequence homology with an amino acid sequence of SEQ ID NO: 3. As used herein, the term "homology" refers to percentage identity between two polynucleotides or two polypeptides as modules. Sequence homology can be measured between one module and another module using a method known in the art. For example, such sequence homology can be measured by virtue of BLAST algorithm.

The expression of a polynucleotide can be enhanced: by substituting or mutating an expression regulatory sequence, introducing a mutation to the sequence of the polynucleotide, and by increasing the copy number of the polynucleotide inserted via a chromosome or introduced with a vector, or a combination thereof, etc.

The expression regulatory sequence of a polynucleotide can be modified. The expression regulatory sequence controls the expression of a polynucleotide operably linked thereto, and can comprise, for example, promoters, terminators, enhancers, silencers and the like. A polynucleotide can have a change in an initiation codon. A polynucleotide can be incorporated into a specific site of a chromosome, thereby increasing copy number. Herein, a specific site can comprise, for example, a transposon site or an intergenic site. In addition, a polynucleotide can be incorporated into an expression vector, and the expression vector is introduced into a host cell, thus increasing copy number.

In an embodiment of the present invention, a polynucleotide, or a polynucleotide having a point mutation is incorporated into a specific site of a chromosome of a microorganism, thus increasing copy number.

In an embodiment of the present invention, a polynucleotide with a promoter sequence, or a polynucleotide having a point mutation with a promoter sequence is incorporated into a specific site of a chromosome of a microorganism, thus overexpressing the nucleic sequence.

In an embodiment of the present invention, a polynucleotide, or a polynucleotide having a point mutation is incorporated into an expression vector, and the expression vector is introduced into a host cell, thus increasing copy number.

In an embodiment of the present invention, a polynucleotide with a promoter sequence, or a polynucleotide having a point mutation with a promoter sequence is incorporated into an expression vector, and the expression vector is introduced into a host cell, thus overexpressing the amino acid sequence.

In a specific embodiment of the present invention, the polynucleotide can comprise a nucleotide sequence of SEQ ID NO: 1.

In an embodiment of the present invention, the polynucleotide encoding an amino acid sequence of SEQ ID NO: 3 has a point mutation such that asparagine at position 520 in the amino acid sequence of SEQ ID NO: 3 is substituted with a different amino acid.

According to the present invention, preferably asparagine at position 520 is substituted with lysine.

According to the present invention, as set forth in SEQ ID NO: 4 is the amino acid sequence following substitution with lysine of asparagine at position 520 in an amino acid sequence set forth in SEQ ID NO: 3.

In an embodiment of the present invention, the polynucleotide sequence having a point mutation is formed from a mutation to the base at position 1560 of a polynucleotide sequence set forth in SEQ ID NO: 1.

According to the present invention, the mutation comprises a mutation of the base at position 1560 of a polynucleotide sequence set forth in SEQ ID NO: 1 from cytosine (C) to adenine (A).

In an embodiment of the present invention, the polynucleotide sequence having a point mutation comprises a polynucleotide sequence set forth in SEQ ID NO: 2.

As used herein, the term "operably linked" refers to a functional link between a regulatory sequence and a polynucleotide sequence, whereby the regulatory sequence controls transcription and/or translation of the polynucleotide sequence. A regulatory sequence can be a strong promoter which can enhance the expression level of a polynucleotide. A regulatory sequence can be a promoter derived from a microorganism belonging to the genus *Corynebacterium* or can be a promoter derived from other microorganisms. For example, the promoter can be trc promoter, gap promoter, tac promoter, T7 promoter, lac promoter, trp promoter, araBAD promoter or cj7 promoter.

In a specific embodiment of the present invention, the promoter is a promoter of a polynucleotide encoding an amino acid sequence of SEQ ID NO: 3 (the gene BBD29_04920).

As used herein, the term "vector" refers to a polynucleotide construct containing a regulatory sequence of a gene and a gene sequence and configured to express a target gene in a suitable host cell. Or, a vector can also refer to a polynucleotide construct containing a sequence for homologous recombination such that due to the vector introduced into a host cell, the regulatory sequence of an endogenous gene in the genome of the host cell can be changed, or a target gene that can be expressed can be inserted into a specific site of the genome of a host. In this regard, the vector used in the present invention can further comprise a selective marker to determine the introduction of the vector into a host cell or the insertion of the vector into the chromosome of a host cell. A selective marker can comprise a marker providing a selectable phenotype, such as drug resistance, auxotroph, resistance to cytotoxic agents, or expression of a surface protein. In an environment treated with such a selective agent, the transformed cell can be selected since only the cell that expresses the selective marker can survive or display different phenotypic traits. The vector as described herein is well known to those skilled in the art, including but not limited to: plasmid, bacteriophage (such as λ bacteriophage or M13 filamentous bacteriophage etc.), cosmid (i.e., Cosmid) or viral vector.

In some specific embodiments of the present invention, the vector used is pK18mobsacB plasmid, and pXMJ19 plasmid.

As used herein, the term "transformation" refers to introduction of a polynucleotide into a host cell, such that the polynucleotide can be replication-competent as an element foreign to a genome or by being inserted into the genome of a host cell. A method for transforming the vector used in the present invention can comprise a method for introducing a nucleic acid into a cell. In addition, as disclosed in the related techniques, a method with electric pulse can be performed according to a host cell.

Herein, the microorganism can be yeast, bacterium, alga or fungi.

According to the present invention, the bacterium can be a microorganism belonging to the genus *Corynebacterium,* such as *Corynebacterium acetoacidophilum, Corynebacterium acetoglutamicum, Corynebacterium callunae, Corynebacterium glutamicum, Brevibacterium flavum, Brevibacterium lactofermentum, Corynebacterium ammoniagenes, Corynebacterium pekinense, Brevibacterium saccharolyticum, Brevibacterium roseum,* and *Brevibacterium thiogenitalis,* etc.

In an embodiment of the present invention, the microorganism belonging to the genus *Corynebacterium* is *Corynebacterium glutamicum* ATCC 13869.

In an embodiment of the present invention, the microorganism belonging to the genus *Corynebacterium* is *Corynebacterium glutamicum* YPGLU001, which yields high production of glutamic acid, and was deposited with Strain Name: *Corynebacterium glutamicum;* Latin Name: *Corynebacteriumglutamicum*; Strain No.: YPGLU001 in China General Microbiological Culture Collection Center, abbreviated as CGMCC, Address: No. 3, Yard 1, Beichen West Road, Chaoyang District, Beijing on November 23, 2020, with Accession No.: CGMCC No. 21220.

According to the present invention, the bacterium can also have additional improvements relating to the increase in the production of L-glutamic acid, such as enhancement or reduction in the activities of, or in the expressions of the genes of glutamate dehydrogenase, glutamine synthetase, glutamine-oxoglutaric acid amino transferase, etc., or substitution of the genes with foreign genes.

A second aspect of the present invention provides a polynucleotide sequence, an amino acid sequence coded by the polynucleotide sequence, a recombinant vector comprising the polynucleotide sequence, and a recombinant strain containing the polynucleotide sequence.

According to the present invention, the polynucleotide sequence comprises a polynucleotide encoding a polypeptide containing an amino acid sequence set forth in SEQ ID NO: 3 with asparagine at position 520 in the sequence substituted with a different amino acid.

According to the present invention, preferably asparagine at position 520 is substituted with lysine.

According to the present invention, as set forth in SEQ ID NO: 4 is the amino acid sequence following substitution with lysine of asparagine at position 520 in an amino acid sequence set forth in SEQ ID NO: 3.

According to the present invention, preferably the polynucleotide encoding a polypeptide containing an amino acid sequence set forth in SEQ ID NO: 3 contains a polynucleotide sequence as set forth in SEQ ID NO: 1.

In an embodiment of the present invention, the polynucleotide sequence is formed from a mutation to the base at position 1560 in a polynucleotide sequence set forth in SEQ ID NO: 1.

According to the present invention, the mutation refers to a change in the base/nucleotide of the site, and the method for mutation can be at least one selected from mutagenesis, PCR site-directed mutation, and/or homologous recombination, etc. In the present invention, PCR site-directed mutation and/or homologous recombination are preferably used.

According to the present invention, the mutation comprises a mutation to the base at position 1560 in a polynucleotide sequence set forth in SEQ ID NO: 1 from cytosine (C) to adenine (A).

In an embodiment of the present invention, the polynucleotide sequence comprises a polynucleotide sequence set forth in SEQ ID NO: 2.

According to the present invention, the amino acid sequence comprises an amino acid sequence set forth in SEQ ID NO: 4.

According to the present invention, the recombinant vector is constructed by introducing the polynucleotide sequence to a plasmid.

In an embodiment of the present invention, the plasmid is pK18mobsacB plasmid.

In another embodiment of the present invention, the plasmid is pXMJ19 plasmid.

Specifically, the polynucleotide sequence and the plasmid can be constructed as a recombinant vector by virtue of NEBuider recombination system.

According to the present invention, the recombinant strain contains the polynucleotide sequence.

As an implementation of the present invention, a starting strain of the recombinant strain is *Corynebacterium glutamicum* CGMCC No. 21220.

As an implementation of the present invention, a starting strain of the recombinant strain is ATCC 13869.

A third aspect of the present invention further provides a method for constructing a recombinant strain generating L-glutamic acid.

According to the present invention, the method for constructing comprises the following step of:
engineering a polynucleotide sequence of a wild-type BBD29_04920 gene as set forth in SEQ ID NO: 1 in a host strain to cause a mutation to the base at position 1560 to provide a recombinant strain containing a mutated BBD29_04920 coding gene.

According to the method for constructing of the present invention, the engineering comprises at least one of mutagenesis, PCR site-directed mutation, and/or homologous recombination, etc.

According to the method for constructing of the present invention, the mutation refers to a change of the base at position 1560 in SEQ ID NO: 1 from cytosine (C) to adenine (A). Specifically, the polynucleotide sequence containing a mutated BBD29_04920 coding gene is set forth in SEQ ID NO: 2.

Further, the method for constructing comprises the steps of:
(1) engineering a nucleotide sequence of a wild-type BBD29_04920 gene as set forth in SEQ ID NO: 1 to cause a mutation to the base at position 1560 to provide a mutated BBD29_04920 gene polynucleotide sequence;
(2) linking the mutated polynucleotide sequence to a plasmid to construct a recombinant vector;
(3) introducing the recombinant vector into a host strain to provide a recombinant strain containing a mutated BBD29 _04920 coding gene.

According to the method for constructing of the present invention, the step (1) comprises constructing a BBD29_04920 gene with a point mutation: according to a genome sequence of an unmodified strain, synthesizing two pairs of primers P1, P2 and P3, P4 for amplifying a fragment of the BBD29_04920 gene, and introducing a point mutation to a wild-type BBD29_04920 gene, SEQ ID NO: 1, by virtue of PCR site-directed mutation to provide a BBD29_04920 gene nucleotide sequence with a point mutation, SEQ ID NO: 2, designated as BBD29_04920^{C1560A}.

In an embodiment of the present invention, the genome of the unmodified strain can be derived from the strain ATCC13869, the genome sequence of which can be available from the NCBI website.

In an implementation of the present invention, in the step (1), the primers are:
P1: 5' CAGTGCCAAGCTTGCATGCCTGCAGGTCGACTCTAG TGTTTCTGTC TTGACCTTGG (SEQ ID NO: 5)
P2: 5' AGCCACGATG GTGACTTTTT GCAAGTTGTT_3' (SEQ ID NO: 6)
P3: 5' AACAACTTGC AAAAAGTCAC CATCGTGGCT 3' (SEQ ID NO: 7)
P4: 5' CAGCTATGACCATGATTACGAATTCGAGCTCGGTACCC ACAGATTGGG CAGGTGCC 3' (SEQ ID NO: 8)

In an implementation of the present invention, the PCR amplification is performed in the following manner: pre-denaturation for 5 min at 94°C, denaturation for 30 s at 94°C, annealing for 30 s at 52°C, and extension for 40 s at 72°C for 30 circles, and overextension for 10 min at 72°C.

In an implementation of the present invention, the overlap PCR amplification is performed in the following manner: pre-denaturation for 5 min at 94°C, denaturation for 30 s at 94°C, annealing for 30 s at 52°C, and extension for 90 s at 72°C for 30 circles, and overextension for 10 min at 72°C.

According to the method for constructing of the present invention, the step (2) comprises constructing a recombinant plasmid, comprising assembling an isolated and purified BBD29_04920^{C1560A} with pK18mobsacB plasmid by virtue of NEBuider recombination system to provide a recombinant plasmid.

According to the method for constructing of the present invention, the step (3) comprises constructing a recombinant strain by transforming a recombinant plasmid to a host strain to provide a recombinant strain.

In an implementation of the present invention, the transforming in the step (3) is by an electrotransformation method.

In an embodiment of the present invention, the host strain is ATCC 13869.

In an embodiment of the present invention, the host strain is *Corynebacterium glutamicum* CGMCC No. 21220.

In an embodiment of the present invention, the recombination is achieved by homologous recombination.

A fourth aspect of the present invention further provides a method for constructing a recombinant strain generating L-glutamic acid.

According to the present invention, the method for constructing comprises the steps of:
amplifying the upstream and downstream homology arm fragments of BBD29_04920 and the sequence of a BBD29_04920 gene coding region and a promoter region thereof, and introducing BBD29_04920 or BBD29_04920^{C1560A} gene into the genome of a host strain in a manner of homologous recombination so as to enable the strain to overexpress the BBD29_04920 or BBD29_04920^{C1560A}gene.

In an embodiment of the present invention, the primers for amplifying the upstream homology arm fragments are:
P7: 5' CAGTGCCAAGCTTGCATGCCTGCAGGTCGACTCTAG GACCCGCTTG CCATACGAAG 3' (SEQ ID NO: 11)
P8: 5' GCATCACAAT GACATAACGA ATCTACTCAT CTGAAGAATC 3' (SEQ ID NO: 12)

In an embodiment of the present invention, the primers for amplifying downstream homologous arm fragments are:
P11: 5' GCGCGGGGAA GCGTCGATAG TTCGTGGGCA CTCTGGTTTG 3' (SEQ ID NO: 15)

P12: 5' CAGCTATGACCATGATTACGAATTCGAGCTCGGTACCC CATAAGAAAC AACCACTTCC
3' (SEQ ID NO: 16)

In an embodiment of the present invention, the primers for amplifying the sequence of the gene coding region and the promoter region thereof are:
P9: 5' GATTCTTCAG ATGAGTAGAT TCGT TATGTCATTG TGATGC 3' (SEQ ID NO: 13)
P10: 5' CAAACCAGAG TGCCCACGAA CTATCGACGC TTCCCCGCGC 3' (SEQ ID NO: 14)

In an embodiment of the present invention, with the above-mentioned P7/P12 as primers again, amplification is performed using as templates a mixture of three fragments, the amplified upstream homology arm fragment, downstream homology arm fragment, and fragment of the sequence of the gene coding region and the promoter region thereof, to provide an integrated homology arm fragment.

In an embodiment of the present invention, the PCR system used comprises: 10 × Ex Taq Buffer 5 µL, dNTP Mixture (2.5 mM each) 4 µL, Mg²⁺ (25 mM) 4 µL, primers (10 pM) 2 µL each, and Ex Taq (5 U/µL) 0.25 µL, in a total volume of 50 µL. The PCR amplification is performed in the following manner: pre-denaturation for 5 min at 94°C, denaturation for 30 s at 94°C, annealing for 30 s at 52°C, extension for 120 s at 72°C (30 circles), and overextension for 10 min at 72°C.

In an embodiment of the present invention, NEBuider recombination system is used to assemble shuttle plasmid PK18mobsacB with the integrated homology arm fragment to provide an integrative plasmid.

In an embodiment of the present invention, a host strain is transfected with the integrative plasmid to introduce BBD29_04920 or BBD29_04920^{C1560A} gene into the genome of the host strain in a manner of homologous recombination.

In an embodiment of the present invention, the host strain is *Corynebacterium glutamicum* CGMCC No. 21220.

In an embodiment of the present invention, the host strain is ATCC 13869.

In an embodiment of the present invention, the host strain is a strain bearing a polynucleotide sequence set forth in SEQ ID NO: 2.

A fifth aspect of the present invention further provides a method for constructing a recombinant strain generating L-glutamic acid.

According to the present invention, the method for constructing comprises the steps of:
amplifying the sequence of BBD29_04920 gene coding region and a promoter region, or the sequence of BBD29_04920^{C1560A} gene coding region and a promoter region, constructing an overexpression plasmid vector, and transforming the vector into a host strain to enable the strain to overexpress BBD29_04920 or BBD29_04920^{C1560A} gene.

In an embodiment of the present invention, the primers for amplifying the sequence of the gene coding region and the promoter region thereof are:
P17: 5' GCTTGCATGCCTGCAGGTCGACTCTAGAGGATCCCCTCGTTATGTCATTGTGATGC 3' (SEQ ID NO: 21)
P18: 5' ATCAGGCTGAAAATCTTCTCTCATCCGCCAAAACCTATCGACGCTTCCCCGCGC 3' (SEQ ID NO: 22).

In an embodiment of the present invention, the PCR system comprises: 10 × Ex Taq Buffer 5 µL, dNTP Mixture (2.5 mM each) 4 µL, Mg²⁺ (25 mM) 4 µL, primers (10 pM) 2 µLeach, Ex Taq (5 U/µL) 0.25 µL, in a total volume of 50 µL. The PCR amplification is performed in the following manner: pre-denaturation for 5 min at 94°C, denaturation for 30 s at 94°C, annealing for 30 s at 52°C, extension for 90 s at 72°C (30 circles), and overextension for 10 min at 72°C.

In an embodiment of the present invention, NEBuider recombination system is used to assemble shuttle plasmid pXMJ19 with BBD29_04920 or BBD29_04920^{C1560A} fragment having its own promoters to provide an overexpression plasmid.

In an embodiment of the present invention, the host strain is *Corynebacterium glutamicum* CGMCC No. 21220.

In an embodiment of the present invention, the host strain is ATCC 13869.

In an embodiment of the present invention, the host strain is a strain bearing a polynucleotide sequence set forth in SEQ ID NO: 2.

The recombinant strain provided in the present invention can be individually applied to fermentation for producing L-glutamic acid, and can also be in hybrid fermentation with other L-glutamic acid-producing bacteria for producing L-glutamic acid.

Another aspect of the present invention provides a method for producing L-glutamic acid, the method comprising culturing the bacterium; and obtaining L-glutamic acid from a culture.

A bacterium can be cultured in a suitable culture medium under culturing conditions as known in the art. The culture medium can comprise: carbon source, nitrogen source, trace elements, and a combination thereof. In culturing, pH of the culture can be adjusted. In addition, bubbles can be prevented from generating in culturing, for example, by using a defoamer to prevent bubbles generation. Further, gases can be injected into the culture in culturing. The gases can comprise any ones capable of maintaining an aerobic condition for the culture. In culturing, the temperature of the culture can be from 20°C to 45°C. The generated L-glutamic acid can be recovered from the culture, i.e., the culture is treated with sulfuric acid or hydrochloric acid, etc. followed by a combination of methods such as anion-exchange chromatography, condensation, crystallization, and isoelectric precipitation.

The present invention also provides a protein, designated as protein BBD29_04920^{N520K}, wherein the protein can be any one of:
A1) a protein whose amino acid sequence is SEQ ID NO: 4;
A2) a protein having 80% or more identity to and the same function as the protein indicated in A1), as obtained by subjecting the amino acid sequence set forth in SEQ ID NO: 4 to substitution and/or deletion and/or addition of amino acid residues;
A3) a fusion protein having the same function, as obtained by linking a tag to the N-terminus and/or C-terminus of A1) or A2).

The present invention also provides a nucleic acid molecule, designated as BBD29_04920^{C1560A}, wherein the nucleic acid molecule BBD29_04920^{C1560A} can be any one of:
B1) a nucleic acid molecule encoding the protein BBD29_04920^{N520K};
B2) a DNA molecule whose coding sequence is set forth in SEQ ID NO: 2;
B3) a DNA molecule whose nucleotide sequence is set forth in SEQ ID NO: 2.

The DNA molecule set forth in SEQ ID NO: 2 is the BBD29_04920^{C1560A} gene of the present invention.

The DNA molecule set forth in SEQ ID NO: 2 (the BBD29_04920^{C1560A} gene) encodes the protein BBD29_04920^{N520K} set forth in SEQ ID NO: 4.

The amino acid sequence of the protein BBD29_04920^{N520K} (SEQ ID NO: 4) is derived from a change of asparagine (N) at position 520 in SEQ ID NO: 3 to lysine (K).

The present invention also provides a biomaterial, wherein the biomaterial can be any one of:
C1) an expression cassette comprising the nucleic acid molecule BBD29_04920^{C1560A} ;
C2) a recombinant vector comprising the nucleic acid molecule BBD29_04920^{C1560A}, or a recombinant vector comprising the expression cassette of C1);
C3) a recombinant microorganism comprising the nucleic acid molecule BBD29_04920^{C1560A}, or a recombinant microorganism comprising the expression cassette of C1), or a recombinant microorganism comprising the recombinant vector of C2).

The present invention also provides any one of the following uses of any one of D1)-D8):
F1) use of any one of D1)-D8) in the regulation of the production of L-glutamic acid of a microorganism;
F2) use of any one of D1)-D8) in the construction of a genetically engineered bacterium for producing L-glutamic acid;
F3) use of any one of D1)-D8) in the preparation of L-glutamic acid;
wherein the D1)-D8) is:
D1) the protein BBD29_04920^{N520K};
D2) the nucleic acid molecule BBD29_04920^{C1560A};
D3) the biomaterial;
D4) a DNA molecule whose nucleotide sequence is SEQ ID NO: 1;
D5) a DNA molecule having 90% or more identity to and the same function as the DNA molecule set forth in SEQ ID NO: 1, as obtained by subjecting the nucleotide sequence set forth in SEQ ID NO: 1 to modification, and/or substitution and/or deletion and/or addition of one or several nucleotides;
D6) an expression cassette comprising the DNA molecule in D4) or D5);
D7) a recombinant vector comprising the DNA molecule in D4) or D5), or a recombinant vector comprising the expression cassette of D6);
D8) a recombinant microorganism comprising the DNA molecule in D4) or D5), or a recombinant microorganism comprising the expression cassette of D6), or a recombinant microorganism comprising the recombinant vector of D7).

The DNA molecule set forth in SEQ ID NO: 1 is the BBD29_04920 gene of the present invention.

The DNA molecule set forth in SEQ ID NO: 1 (the BBD29_04920 gene) encodes a protein set forth in SEQ ID NO: 3.

Herein, identity refers to the identity between amino acid sequences or nucleotide sequences. International homology retrieval sites on the Internet can be employed to determine the identity between amino acid sequences or nucleotide sequences, such as the BLAST page on the NCBI homepage website. For example, in Advanced BLAST2.1, blastp can be used as a program, with the Expect value set at 10, each Filter set to OFF, BLOSUM62 used as Matrix, and Gap existence cost, Per residue gap cost and Lambda ratio set at 11, 1 and 0.85 (default values), respectively, and a search is done for the identity between a pair of amino acid sequences for calculation, and then a value of identity (%) can be available.

Herein, the 80% or more identity can be an identity of at least 80%, 81%, 82%, 83%, 84%, 85%, 86%, 87%, 88%, 89%, 90%, 91%, 92%, 93%, 94%, 95%, 96%, 97%, 98% or 99%.

Herein, the 90% or more identity can be an identity of at least 90%, 91%, 92%, 93%, 94%, 95%, 96%, 97%, 98% or 99%.

The regulation of the production of L-glutamic acid of a microorganism as described herein can be increasing or reducing the amount of the accumulation of L-glutamic acid in the microorganism (i.e., promoting or inhibiting the biosynthesis of L-glutamic acid).

The present invention also provides a method for increasing the production of L-glutamic acid in a microorganism, the method comprising any one of:
E1) increasing the expression amount, or content of the nucleic acid molecule BBD29_04920^{C1560A} in a target microorganism to provide a microorganism having a greater production of L-glutamic acid than the target microorganism;
E2) increasing the expression amount, or content of the DNA molecule of D4) or D5) in a target microorganism to provide a microorganism having a greater production of L-glutamic acid than the target microorganism;
E3) performing a mutation on the DNA molecule whose nucleotide sequence is SEQ ID NO: 1 in the target microorganism to provide a microorganism having a greater production of L-glutamic acid than the target microorganism.

In the method above, the mutation can be a point mutation, i.e., a mutation of a single nucleotide.

In the method above, the point mutation can be a mutation of asparagine residue at position 520 in an amino acid sequence coded by the DNA molecule set forth in SEQ ID NO: 1 to another amino acid residue.

In the method above, the point mutation can be a mutation of asparagine at position 520 in an amino acid sequence coded by the DNA molecule set forth in SEQ ID NO: 1 to lysine, providing a mutated protein BBD29_04920^{N520K} whose amino acid sequence is SEQ ID NO: 4.

The mutation refers to a change in one or several bases in a gene through site-directed mutation, resulting in a change in the constitution of the amino acids of a corresponding protein, thereby generating a new protein or causing a new function to the original protein, that is, site-directed mutation to genes. Techniques for site-directed mutation to genes such as oligonucleotide primer-mediated site-directed mutation, PCR-mediated site-directed mutation, or cassette mutation are well known to those skilled in the art.

The point mutation as described herein can be replacement of a single base, insertion of a single base, or deletion of a single base, and specifically, replacement of a single base. The replacement of a single base can be allelic replacement.

The point mutation can be performing a nucleic acid modification to cytosine (C) at position 1560 of the BBD29_04920 gene (SEQ ID NO: 1).

Specifically, the point mutation can be performing a mutation to cytosine (G) at position 1560 of the BBD29_04920 gene (SEQ ID NO: 1) to adenine (A) to provide a DNA molecule set forth in SEQ ID NO: 2.

Herein, the recombinant vector can be, specifically, recombinant vector pK18-BBD29_04920^{C1560A}, PK18mobsacB-BBD29_04920, PK18mobsacB-BBD29_04920^{C1560A}, pXMJ19-BBD29_04920 or pXMJ19-BBD29_04920^{C1560A}.

The recombinant vector pK18-BBD29_04920^{C1560A} contains a DNA molecule set forth as positions 846-1788 in the mutated gene BBD29_04920^{C1560A} as set forth in SEQ ID NO: 2.

The recombinant vector PK18mobsacB-BBD29_04920 is used to integrate an exogenous gene BBD29_04920 into a chromosome of a host so as to overexpress a wild-type BBD29_04920 gene in a producer bacterium.

The recombinant vector pK18mobsacB-BBD29_04920^{C1560A} is used to integrate an exogenous gene BBD29_04920^{C1560A} into a chromosome of a host so as to overexpress a mutant-type BBD29_04920^{C1560A} gene in a producer bacterium.

The recombinant vector pXMJ19-BBD29_04920 is used to express via a plasmid an exogenous gene BBD29_04920 outside a chromosome so as to overexpress a wild-type BBD29_04920 gene in a producer bacterium.

The recombinant vector pXMJ19-BBD29_04920^{C1560A} is used to express via a plasmid an exogenous gene BBD29_04920^{C1560A} outside a chromosome so as to overexpress a mutant-type gene BBD29_04920^{C1560A} in a producer bacterium.

The recombinant vectors pK18-BBD29_04920^{C1560A}, PK18mobsacB-BBD29_04920, PK18mobsacB-BBD29_04920^{C1560A,} pXMJ19-BBD29_04920 and pXMJ19-BBD29_04920^{C1560A} are all within the scope of protection of the present invention.

Herein, the recombinant microorganism can be, specifically, a recombinant bacterium YPG-001, YPG-002, YPG-003, YPG-004 or YPG-005.

The recombinant bacterium YPG-001 is a recombinant bacterium obtained by transformation of the recombinant vector pK18-BBD29_04920^{C1560A} into *Corynebacterium glutamicum* CGMCC No. 21220, and the recombinant bacterium YPG-001 contains the mutated gene BBD29_04920^{C1560A} set forth in SEQ ID NO: 2.

The recombinant bacterium YPG-002 contains a double-copy BBD29_04920 gene set forth in SEQ ID NO: 1; a recombinant bacterium containing a double-copy BBD29_04920 gene can significantly and steadily increase the expression amount of BBD29_04920 gene. The recombinant bacterium YPG-002 is an engineered bacterium overexpressing a wild-type BBD29_04920 gene on genome.

The recombinant bacterium YPG-003 contains a mutated BBD29_04920^{C1560A} gene set forth in SEQ ID NO: 2; the recombinant bacterium YPG-003 is an engineered bacterium overexpressing a mutant-type BBD29_04920^{C1560A} gene on genome.

The recombinant bacterium YPG-004 contains a double-copy BBD29_04920 gene set forth in SEQ ID NO: 1; the recombinant bacterium YPG-004 is an engineered bacterium overexpressing a wild-type BBD29_04920 gene on a plasmid, i.e., overexpression outside a chromosome via a plasmid pXMJ19-BBD29_04920.

The recombinant bacterium YPG-005 contains a mutated BBD29_04920^{C1560A} gene set forth in SEQ ID NO: 2; the recombinant bacterium YPG-005 is an engineered bacterium overexpressing a mutant-type BBD29_04920^{C1560A} gene on a plasmid, i.e., overexpression outside a chromosome via a plasmid pXMJ19-BBD29_04920^{C1560A}.

The recombinant bacteria YPG-001, YPG-002, YPG-003, YPG-004 or YPG-005 are all within the scope of protection of the present invention.

The present invention also provides a method for constructing the recombinant microorganism, the method comprising at least one of:
F1) introducing the nucleic acid molecule BBD29_04920^{C1560A} into a target microorganism to provide the recombinant microorganism;
F2) introducing the DNA molecule set forth in SEQ ID NO: 1 into a target microorganism to provide the recombinant microorganism;
F3) editing the DNA molecule set forth in SEQ ID NO: 1 with a gene editing measure (such as single-base gene editing) to contain the DNA molecule set forth in SEQ ID NO: 2 in a target microorganism.

The introducing can be host bacterium transformation with a vector bearing a DNA molecule of the present invention through any of known transformation methods, such as a chemical transformation method or an electrotransformation method. The introduced DNA molecule can be single-copy, or multi-copy. The introducing can be integration of an exogenous gene into a chromosome of a host, or expression outside a chromosome via a plasmid.

The present invention also provides a method for preparing L-glutamic acid, the method comprising producing L-glutamic acid with any one of the recombinant microorganisms as described herein.

In the method above, the method can be preparation of L-glutamic acid in a fermentation method, and the recombinant microorganism can be the genus *Corynebacterium,* and specifically, *Corynebacterium glutamicum* and variants thereof.

In the present invention:
SEQ ID NO: 1: sequence of BBD29_04920 wild type coding region 
ATGACTACATCTGACCCAAATTCGAAACCGATAGTGGAGGATGCTCAGCCAGAGCAGATCAC
CGCAACCGAAGAACTGGCGGGTTTGCTTGAGAATCCAACTAACCTGGAAGGGAAACTGGCCG
ACGCCGAAGAGGAAATTATCCTCGAAGGCGAAGACGCCCAGGCCTCACTTAACTGGTCAGTC
ATCGTTCCAGCCCTAGTCATTGTCCTAGCGACAGTGGTGTGGGGTATCGGATTCAAAGATAGC
TTTACCAACTTTGCTAGTTCTGCGTTGTCAGCAGTAGTTGACAATCTCGGCTGGGCCTTCATTT
TGTTTGGCACAGTCTTTGTATTTTTTATCGTTGTTATCGCCGCTAGTAAATTCGGCACGATTCG
CTTAGGCCGCATTGATGAAGCACCAGAGTTTCGCACGGTGTCATGGATTTCCATGATGTTTGC
TGCAGGTATGGGTATTGGTTTGATGTTCTACGGAACCACAGAACCTTTAACCTTCTACCG
CAATGGTGTACCTGGATATGATGAACACAATGTTGGCGTTGCTATGTCCA
CGACAATGTTCCACTGGACCTTGCATCCATGGGCTATCTACGCAATTGTGGGCCTAGCCATTG
CCTATTCGACCTTCCGAGTGGGCCGTAAACAGCTTCT
AAGCTCTGCATTCGTGCCACTCATTGGTGAAAAAGGTGCAGAAGGATGGT
TGGGCAAGCTCATCGACATCCTGGCGATTATCGCCACAGTATTCGGCACC
GCATGTTCCCTTGGCCTGGGTGCGCTGCAGATCGGTGCAGGACTTTCCGC
AGCCAACATCATTGAAAATCCGAGTGACTGGACTGTCATTGGTATTGTTT
CTGTCTTGACCTTGGCATTTATCTTCTCTGCTATTTCTGGTGTGGGCAAGGGAATCCAGTACCT
CTCCAACGCCAACATGGTTCTGGCAGCTCTGCTCGCGATTTTCGTGTTCGTTGTCGGACCAAC
CGTGTCGATTTTGAACCTGCTGCCAGGTTCTATTGGCAACTACCTGTCCAACTTCTTCCAAATG
GCAGGCCGCACTGCCATGAGTGCCGACGGCACAGCAGGTGAGTGGCTAGGTAGCTGGACCAT
CTTCTACTGGGCATGGTGGATCTCTTGGTCACCATTCGTAGGAATGTTCTTGGCACGTATTTCC
CGTGGCCGCTCCATCCGTGAGTTCATCCTGGGCGTGTTGCTCGTCCCAGCAGGTGTGTCCACC
GTATGGTTCTCCATTTTTGGTGGCACTGCGATTGTCTTCGAACAAAATGGGGAATCCATTTGG
GGTGATGGTGCAGCAGAAGAGCAGCTCTTTGGATTGCTTCATGCACTTCCAGGTGGGCAAAT
AATGGGCATCATCGCCATGATTTTGCTGGGTACTTTCTTCATTACCTCTGCAGACTCTGCTTCC
ACCGTCATGGGCACCATGAGTCAGCACGGCCAGCTGGAAGCCAACAAGTGGGTGACAGCTGC
 
CTGGGGTGTTGCTACCGCAGCTATTGGACTAACGCTATTGCTTTCTGGTGGTGACAATGCCTT
GAACAACTTGCAAAACGTCACCATCGTGGCTGCAACACCATTCCTGTTTGTGGTTATTGGATT
GATGTTTGCGTTAGTCAAGGACTTAAGCAATGATGTGATCTACCTCGAGTACCGTGAGCAGCA
ACGCTTCAACGCGCGCCTTGCCCGTGAACGTCGTGTTCACAATGAACACCGCAAGCGTGAAC
TGGCTGCAAAGCGACGCAGGGAGCGTAA GGCGAGTGGC GCGGGGAAGCGTCGATAG
SEQ ID NO: 2: sequence ofBBD29_04920^{C1560A} coding region 
ATGACTACATCTGACCCAAATTCGAAACCGATAGTGGAGGATGCTCAGCCAGAGCAGATCAC
CGCAACCGAAGAACTGGCGGGTTTGCTTGAGAATCCAACTAACCTGGAAGGGAAACTGGCCG
ACGCCGAAGAGGAAATTATCCTCGAAGGCGAAGACGCCCAGGCCTCACTTAACTGGTCAGTC
ATCGTTCCAGCCCTAGTCATTGTCCTAGCGACAGTGGTGTGGGGTATCGGATTCAAAGATAGC
TTTACCAACTTTGCTAGTTCTGCGTTGTCAGCAGTAGTTGACAATCTCGGCTGGGCCTTCATTT
TGTTTGGCACAGTCTTTGTATTTTTTATCGTTGTTATCGCCGCTAGTAAATTCGGCACGATTCG
CTTAGGCCGCATTGATGAAGCACCAGAGTTTCGCACGGTGTCATGGATTTCCATGATGTTTGC
TGCAGGTATGGGTATTGGTTTGATGTTCTACGGAACCACAGAACCTTTAACCTTCTACCG
CAATGGTGTACCTGGATATGATGAACACAATGTTGGCGTTGCTATGTCCA
CGACAATGTTCCACTGGACCTTGCATCCATGGGCTATCTACGCAATTGTGGGCCTAGCCATTG
CCTATTCGACCTTCCGAGTGGGCCGTAAACAGCTTCT
AAGCTCTGCATTCGTGCCACTCATTGGTGAAAAAGGTGCAGAAGGATGGT
TGGGCAAGCTCATCGACATCCTGGCGATTATCGCCACAGTATTCGGCACC
GCATGTTCCCTTGGCCTGGGTGCGCTGCAGATCGGTGCAGGACTTTCCGC
AGCCAACATCATTGAAAATCCGAGTGACTGGACTGTCATTGGTATTGTTT
CTGTCTTGACCTTGGCATTTATCTTCTCTGCTATTTCTGGTGTGGGCAAGGGAATCCAGTACCT
CTCCAACGCCAACATGGTTCTGGCAGCTCTGCTCGCGATTTTCGTGTTCGTTGTCGGACCAAC
CGTGTCGATTTTGAACCTGCTGCCAGGTTCTATTGGCAACTACCTGTCCAACTTCTTCCAAATG
GCAGGCCGCACTGCCATGAGTGCCGACGGCACAGCAGGTGAGTGGCTAGGTAGCTGGACCAT
CTTCTACTGGGCATGGTGGATCTCTTGGTCACCATTCGTAGGAATGTTCTTGGCACGTATTTCC
CGTGGCCGCTCCATCCGTGAGTTCATCCTGGGCGTGTTGCTCGTCCCAGCAGGTGTGTCCACC
GTATGGTTCTCCATTTTTGGTGGCACTGCGATTGTCTTCGAACAAAATGGGGAATCCATTTGG
GGTGATGGTGCAGCAGAAGAGCAGCTCTTTGGATTGCTTCATGCACTTCCAGGTGGGCAAAT
AATGGGCATCATCGCCATGATTTTGCTGGGTACTTTCTTCATTACCTCTGCAGACTCTGCTTCC
ACCGTCATGGGCACCATGAGTCAGCACGGCCAGCTGGAAGCCAACAAGTGGGTGACAGCTGC
CTGGGGTGTTGCTACCGCAGCTATTGGACTAACGCTATTGCTTTCTGGTGGTGACAATGCCTT
GAACAACTTGCAAAAAGTCACCATCGTGGCTGCAACACCATTCCTGTTTGTGGTTATTGGATT
GATGTTTGCGTTAGTCAAGGACTTAAGCAATGATGTGATCTACCTCGAGTACCGTGAGCAGCA
ACGCTTCAACGCGCGCCTTGCCCGTGAACGTCGTGTTCACAATGAACACCGCAAGCGTGAAC
TGGCTGCAAAGCGACGCAGGGAGCGTAA GGCGAGTGGC GCGGGGAAGCGTCGATAG
SEQ ID NO: 3: amino acid sequence of protein coded by BBD29_04920 wild type 
MTTSDPNSKP IVEDAQPEQI TATEELAGLL ENPTNLEGKL ADAEEEIILE GEDAQASLNW
SVIVPALVIV LATVVWGIGF KDSFTNFASS ALSAVVDNLG WAFILFGTVF VFFIVVIAAS
KFGTIRLGRI DEAPEFRTVS WISMMFAAGM GIGLMFYGTT EPLTFYRNGV PGYDEHNVGV
AMSTTMFHWT LHPWAIYAIV GLAIAYSTFR VGRKQLLSSA FVPLIGEKGA EGWLGKLIDI
LAIIATVFGT ACSLGLGALQ IGAGLSAANI IENPSDWTVI GIVSVLTLAF IFSAISGVGK
GIQYLSNANM VLAALLAIFV FVVGPTVSIL NLLPGSIGNY LSNFFQMAGR TAMSADGTAG
EWLGSWTIFY WAWWISWSPF VGMFLARISR GRSIREFILG VLLVPAGVST VWFSIFGGTA
IVFEQNGESI WGDGAAEEQL FGLLHALPGG QIMGIIAMIL LGTFFITSAD SASTVMGTMS
QHGQLEANKW VTAAWGVATA AIGLTLLLSG GDNALNNLQN VTIVAATPFL FVVIGLMFAL
 VKDLSNDVIY LEYREQQRFN ARLARERRVH NEHRKRELAA KRRRERKASG AGKRR
SEQ ID NO: 4: amino acid sequence of protein coded by BBD29_04920^{N520K} 
MTTSDPNSKP IVEDAQPEQI TATEELAGLL ENPTNLEGKL ADAEEEIILE GEDAQASLNW
SVIVPALVIV LATVVWGIGF KDSFTNFASS ALSAVVDNLG WAFILFGTVF VFFIVVIAAS
KFGTIRLGRI DEAPEFRTVS WISMMFAAGM GIGLMFYGTT EPLTFYRNGV PGYDEHNVGV
AMSTTMFHWT LHPWAIYAIV GLAIAYSTFR VGRKQLLSSA FVPLIGEKGA EGWLGKLIDI
LAIIATVFGT ACSLGLGALQ IGAGLSAANI IENPSDWTVI GIVSVLTLAF IFSAISGVGK
GIQYLSNANM VLAALLAIFV FVVGPTVSIL NLLPGSIGNY LSNFFQMAGR TAMSADGTAG
EWLGSWTIFY WAWWISWSPF VGMFLARISR GRSIREFILG VLLVPAGVST VWFSIFGGTA
IVFEQNGESI WGDGAAEEQL FGLLHALPGG QIMGIIAMIL LGTFFITSAD SASTVMGTMS
QHGQLEANKW VTAAWGVATA AIGLTLLLSG GDNALNNLQK VTIVAATPFL FVVIGLMFAL
VKDLSNDVIY LEYREQQRFN ARLARERRVH NEHRKRELAA KRRRERKASG AGKRR
 Depositary Information: Strain Name: *Corynebacterium glutamicum;* Latin Name: *Corynebacterium glutamicum;* Strain No.: YPGLU001; Depositary Institution: China General Microbiological Culture Collection Center, abbreviated as CGMCC; Address: No. 3, Yard 1, Beichen West Road, Chaoyang District, Beijing; Depositary Date: November 23, 2020; Accession No. in the depositary center: CGMCC No. 21220.

### Best Modes for Carrying out the Invention

The technical solution of the present invention will be further described in detail in connection to specific examples. It should be understood that the following examples are given only to illustrate and explain the present invention, and it should not be interpreted as limitation to the scope of protection of the present invention. All techniques implemented based on the above content of the present invention are within the scope intended to be protected by the present invention. Unless otherwise specified specially, the raw materials and reagents used in the following examples can be commercially available or can be prepared by known methods; all the operations carried out are known in the art, or carried out according to the user manual of the commercially available products.

In the following examples, the constitution of the basic medium used to culture the strain is the same, and sucrose, kanamycin or chloramphenicol are added based on the constitution of the basic medium for a corresponding demand. The components in the basic medium are listed as follows, which provide the basic medium upon being dissolved in water:

| Ingredients | Formulation |
|---|---|
| Sucrose | 10 g/L |
| Polypeptone | 10 g/L |
| Beef Extract | 10 g/L |
| Powdered Yeast | 5 g/L |
| Urea | 2 g/L |
| Sodium Chloride | 2.5 g/L |
| Powdered Agar | 20 g/L |
| pH of Culture Medium and Temperature for Culturing Strains | |
| pH | 7.0 |
| Temperature for Culturing | 32 °C |

*Corynebacterium glutamicum* YPGLU001 CGMCC No. 21220 in the following examples has been deposited at China General Microbiological Culture Collection Center (abbreviated as CGMCC, Address: No. 3, Yard 1, Beichen West Road, Chaoyang District, Beijing, Institute of Microbiology, Chinese Academy of Sciences) on November 23, 2020, with Accession No. CGMCC No. 21220. *Corynebacterium glutamicum*YPGLU001 is also known as *Corynebacterium glutamicum* CGMCC No. 21220.

### Example 1. Construction of Transformation Vector pK18-BBD29_04920^{C1560A} Comprising BBD29_04920 Gene Coding Region with Point Mutation

According to the genome sequence of *Corynebacterium glutamicum* ATCC13869 published on NCBI, two pairs of primers for amplifying the sequence of BBD29_04920 gene coding region (SEQ ID NO: 1 BBD29_04920) are designed and synthesized, and a point mutation is introduced into a strain, *Corynebacterium glutamicum* CGMCC No. 21220, in allelic replacement (the BBD29_04920 gene coding region on the chromosome of the strain is confirmed consistent with that of ATCC13869 upon sequencing). The amino acid sequence corresponding to a coded protein is SEQ ID NO: 3, with a change of cytosine (C) at position 1560 in the nucleotide sequence of BBD29_04920 gene to adenine (A) (SEQ ID NO: 2: BBD29_04920^{C1560A}), and thus a change of asparagine (N) at position 520 in the amino acid sequence corresponding to the coded protein to lysine (K) (SEQ ID NO: 4: BBD29_04920^{N520K}). The primers are designed as follows (synthesized by Invitrogen Corporation, Shanghai):
P1: 5' CAGTGCCAAGCTTGCATGCCTGCAGGTCGACTCTAG TGTTTCTGTC TTGACCTTGG (SEQ ID NO: 5)
P2: 5' AGCCACGATG GTGACTTTTT GCAAGTTGTT_3' (SEQ ID NO: 6)
P3: 5' AACAACTTGC AAAAAGTCAC CATCGTGGCT 3' (SEQ ID NO: 7)
P4: 5' CAGCTATGACCATGATTACGAATTCGAGCTCGGTACCC ACAGATTGGG CAGGTGCC 3' (SEQ ID NO: 8)

Method for Construction: using *Corynebacterium glutamicum* ATCC13869 as a template, PCR amplification is performed with respective primers P1, P2 and P3, P4.

PCR System:10×Ex Taq Buffer 5 µL, dNTP Mixture (2.5 mM each) 4 µL, Mg²⁺ (25mM) 4 µL, primers (10pM) 2 µL each, Ex Taq (5U/µL) 0.25 µL, a template 1 µL, and water as balance in a total volume of 50 µL.

The above PCR amplification is performed by pre-denaturation for 5min at 94°C, denaturation for 30s at 94°C, annealing for 30s at 52°C, and extension for 40s at 72°C for 30 circles, and overextension for 10min at 72°C, to provide two DNA fragments containing a BBD29_04920^{C1560A} gene coding region each sized in 766 bp and 778 bp (BBD29_04920 Up and BBD29_04920 Down).

The above two DNA fragments are isolated via Agarose Gel Electrophoresis and purified. The above two DNA fragments are then used as a template with P1 and P4 as primers for overlap PCR amplification to provide a fragment in 1514bp in length (that is BBD29_04920^{C1560A}-up-down, whose nucleotide sequence is positions 846-1788 of SEQ ID NO: 2.

PCR System:10×Ex Taq Buffer 5 µL, dNTP Mixture (2.5 mM each) 4 µL, Mg²⁺ (25mM) 4 µL, primers (10pM) 2 µL each, Ex Taq (5U/µL) 0.25 µL, a template 1 µL, and water as balance in a total volume of 50 µL.

The above overlap PCR amplification is performed by pre-denaturation for 5min at 94°C, denaturation for 30s at 94°C, annealing for 30s at 52°C, and extension for 90s at 72°C for 30 circles, and overextension for 10min at 72°C.

The DNA fragment causes a change of cytosine (C) at position 1560 in the BBD29_04920 gene coding region in *Corynebacterium glutamicum* CGMCC No. 21220 to adenine (A), and finally resulting in a change of the amino acid at position 520 of a coded protein from asparagine (N) to lysine (K).

After pK18mobsacB plasmid (purchased from Addgene Coporation) is cleaved with *Xba I,* BBD29_04920^{C1560A} and the linearized pK18mobsacB plasmid are isolated via Agarose Gel Electrophoresis and purified, followed by assembly using NEBuider recombination system (NEB E5520S) to provide vector pK18-BBD29_04920^{C1560A}, a plasmid containing a kanamycin-resistant marker. And, the vector pK18-BBD29_04920^{C1560A} is sent for sequencing and identification in a company for sequencing, while vector pK18-BBD29_04920^{C1560A} containing a correct point mutation (C-A) is preserved for reserve.

pK18-BBD29_04920^{cl560A} is a recombinant vector obtained by inserting the DNA fragment BBD29_04920^{C1560A}-up-down set forth as positions 846-1488 of SEQ ID NO: 2 in the Sequence Listing into the XbaI recognition sites in pK18mobsacB vector, while keeping the other sequences of the pK18mobsacB vector unchanged.

Example 2. Construction of Engineered Strain Comprising BBD29_04920^{C1560A} with Point Mutation Method for Construction: Plasmid pK18-BBD29_04920^{cl560A} following allelic replacement is electrotransformed into *Corynebacterium glutamicum* CGMCC No. 21220. The cultured mono-colonies are each identified with primer P1 and universal primer M13R (5' CAG GAA ACA GCT ATG ACC 3') for a positive strain with a band in about 1521 bp (as set forth in SEQ ID NO: 29 for the sequence) amplified therefrom. The positive strain is cultured on a culture medium containing 15% sucrose, and the cultured mono-colonies are cultured on culture media with and without kanamycin, respectively. Strains that grow on a culture medium without kanamycin and do not grow on a culture medium with kanamycin are further subjected for PCR identification with the primers as follows (synthesized by Invitrogen Corporation, Shanghai):
P5: 5' CTATTGCTTT CTGGTGGTG 3' (SEQ ID NO: 9)
P6: 5' TCGCCTTACG CTCCCTGCGT 3' (SEQ ID NO: 10)

The above amplified products from PCR (as set forth in SEQ ID NO: 30 for the sequence) are subjected to denaturation at a high temperature and ice bath before SSCP electrophoresis (with an amplified fragment of plasmid pK18-BBD29_04920^{cl560A} as a positive control, an amplified fragment of ATCC13869 as a negative control, and water as a blank control). Since fragments are different in their structures, their locations in electrophoresis are different. Thus, a strain having successful allelic replacement is such a strain that the location of its fragment in electrophoresis is inconsistent with that of a negative control fragment and consistent with that of a positive control fragment. Primers P5 and P6 are used again for PCR amplification of a target fragment of the strain with successful allelic replacement, which is then ligated to PMD19-T vector for sequencing. By virtue of sequence alignment, the sequence with a mutated base sequence verified that the allelic replacement of the strain is successful, which is designated as YPG-001.

Recombinant bacterium YPG-001 is a genetically engineered strain YPG-001 with a point mutation (C-A), as obtained by introducing a point mutation C1560A to a BBD29_04920 gene coding region (SEQ ID NO: 1) of *Corynebacterium glutamicum* CGMCC No. 21220 in allelic replacement to cause a mutation at position 1560 of the gene from C to A, while keeping the other sequences of the gene unchanged.

Compared with *Corynebacterium glutamicum* CGMCC21220, *Corynebacterium glutamicum* YPG-001 only differs in the substitution of the BBD29_04920 gene set forth in SEQ ID NO:1 in the genome of *Corynebacterium glutamicum* CGMCC21220 with a BBD29_04920^{C1560A} gene set forth in SEQ ID NO:2. There is only one nucleotide difference between SEQ ID NO: 1 and SEQ ID NO: 2, located at position 1560.

Preparation of PAGE and Conditions for SSCP Electrophoresis are as follows:

| Ingredients | Amount (Final Concentration of 8% for Acrylamide Formulation) |
|---|---|
| 40% Acrylamide | 8 mL |
| ddH₂O | 26 mL |
| Glycerol | 4 mL |
| 10×TBE | 2 mL |
| TEMED | 40 µL |
| 10%AP | 600 µL |
| Conditions for Electrophoresis | Electrophoresis Tank Placed into Ice, 1 ×TBE Buffer, Voltage 120V for 10h |

### Example 3. Construction of Engineered Strain with BBD29_04920 or BBD29_04920^{C1560A} Gene Overexpressing on Genome

According to the genome sequence of *Corynebacterium glutamicum* ATCC13869 published on NCBI, three pairs of primers are designed and synthesized for amplification of upstream and downstream homology arm fragments, and a sequence of BBD29_04920 gene coding region and a promoter region for introduction of BBD29_04920 or BBD29_04920^{C1560A} gene into the strain *Corynebacterium glutamicum* CGMCC No. 21220 through homologous recombination.

The primers are designed as follows (synthesized by Invitrogen Corporation, Shanghai):
P7: 5' CAGTGCCAAGCTTGCATGCCTGCAGGTCGACTCTAG GACCCGCTTG CCATACGAAG 3' (SEQ ID NO: 11)
P8: 5' GCATCACAAT GACATAACGA ATCTACTCAT CTGAAGAATC 3' (SEQ ID NO: 12)
P9: 5' GATTCTTCAG ATGAGTAGAT TCGT TATGTCATTG TGATGC 3' (SEQ ID NO: 13)
P10: 5' CAAACCAGAG TGCCCACGAA CTATCGACGC TTCCCCGCGC 3' (SEQ ID NO: 14)
P11: 5' GCGCGGGGAA GCGTCGATAG TTCGTGGGCA CTCTGGTTTG 3' (SEQ ID NO: 15)

P12: 5' CAGCTATGACCATGATTACGAATTCGAGCTCGGTACCC CATAAGAAAC AACCACTTCC
3' (SEQ ID NO: 16)

Method for Construction: Using *Corynebacterium glutamicum* ATCC13869 or YPG-001, respectively, as a template, PCR amplification is performed with primers P7/P8, P9/P10, and P11/P22, respectively, to provide an upstream homologous arm fragment in about 806bp (as set forth in SEQ ID NO: 31 for the sequence), a fragment of BBD29_04920 or BBD29_04920^{C1560A} gene coding region and promoter region in about 1987bp (as set forth in SEQ ID NO: 32 or SEQ ID NO: 33 for the sequence), and a downstream homologous arm fragment in about 788bp (as set forth in SEQ ID NO: 34 for the sequence). Further, using P7/P12 as primers, amplification is performed with a mixture of the three amplified fragments above as a template to provide integrated homologous arm fragment upstream-BBD29_04920-downstream (as set forth in SEQ ID NO: 35 for the sequence); or integrated homologous arm fragment upstream-BBD29_04920^{C1560A}-downstream (as set forth in SEQ ID NO: 36 for the sequence). After the PCR reaction is completed, electrophoresis is performed on the amplified products for recovering a desired DNA fragment in about 3501bp using a Column DNA Gel Recovery Kit (TIANGEN), which is ligated, using NEBuider recombination system, to shuttle plasmid PK18mobsacB having been cleaved with *Xba* I and recovered to provide an integrative plasmid PK18mobsacB-BBD29_04920 or PK18mobsacB-BBD29_04920^{C1560}A. The plasmid contains a kanamycin-resistant marker through which a recombinant having a plasmid integrated into genome can be obtained by screening with kanamycin.

pK18mobsacB-BBD29_04920 is a recombinant vector obtained by inserting the integrated homologous arm fragment upstream-BBD29_04920-downstream into the Xba I recognition sites of the shuttle plasmid pk18mobsacB.

pK18mobsacB-BBD29_04920^{C1560A} is a recombinant vector obtained by inserting the integrated homologous arm fragment upstream-BBD29_04920^{C1560A}-downstream into the Xba I recognition sites of the shuttle plasmid pk18mobsacB.

PCR System: 10×Ex Taq Buffer 5 µL, dNTP Mixture (2.5 mM each) 4 µL, Mg²⁺ (25mM) 4 µL, primers (10pM) 2 µL each, Ex Taq (5U/µL) 0.25 µL, a template 1 µL, and water as balance in a total volume of 50 µL.

The PCR amplification is performed by pre-denaturation for 5min at 94°C, denaturation for 30s at 94°C, annealing for 30s at 52°C, and extension for 120s at 72°C (30 cycles), and overextension for 10min at 72°C.

The two integrative plasmids are each introduced into the strain *Corynebacterium glutamicum* CGMCC No. 21220 by virtue of electrotransformation, and PCR identification is performed on the cultured mono-colonies with primers P13/P14 for a positive strain containing a fragment in about 1674 bp (as set forth in SEQ ID NO: 37 for the sequence) from PCR amplification, while a strain without any fragments amplified therefrom is an original one. Having being screened with 15% sucrose, the positive strain is cultured on culture media with and without kanamycin, respectively, and strains that grow on a culture medium without kanamycin and do not grow on a culture medium with kanamycin are further subjected to PCR for identification with primers P15/P16. The strain having a band in about 1943 bp (as set forth in SEQ ID NO: 38 for the sequence) amplified therefrom is a strain having BBD29_04920 or BBD29_04920^{C1560A} gene integrated into the genome of *Corynebacterium glutamicum* CGMCC No. 21220, designated as YPG-002 (without a mutation point) and YPG-003 (with a mutation point).
P13: 5' GTCCAAGGTG ACGGCCGCAC 3' (SEQ ID NO: 17)
P14: 5' CTTTTTCACC AATGAGTGGC 3' (SEQ ID NO: 18)
P15: 5' CCGTAAACAG CTTCTAAGCT 3' (SEQ ID NO: 19)
P16: 5' ATATTCGGCC CAGCAGCAGC 3' (SEQ ID NO: 20)

The recombinant bacterium YPG-002 is a recombinant bacterium containing a double-copy BBD29_04920 gene set forth in SEQ ID NO: 1, as obtained by integrating the integrated homologous arm fragment upstream-BBD29_04920-downstream into the genome of the strain *Corynebacterium glutamicum* YPGLU001, and the recombinant bacterium containing a double-copy BBD29_04920 gene can significantly and steadily increase the expression amount of BBD29_04920.

The recombinant bacterium YPG-003 is a recombinant bacterium containing a BBD29_04920^{C1560A} mutant gene set forth in SEQ ID NO: 2, as obtained by integrating the integrated homologous arm fragment upstream-BBD29_04920^{C1560A}-downstream into the genome of the strain *Corynebacterium glutamicum* YPGLU001.

### Example 4. Construction of Engineered Strain Overexpressing BBD29_04920 or BBD29_04920^{C1560A} Gene on Plasmid

According to the genome sequence of *Corynebacterium glutamicum* ATCC13869 published on NCBI, a pair of primers for amplifying a sequence of BBD29_04920 gene coding region and promoter region are designed and synthesized. The primers are designed as follows (synthesized by Invitrogen Corporation, Shanghai):
P17: 5' GCTTGCATGCCTGCAGGTCGACTCTAGAGGATCCCCTCGTTATGTCATTGTGATGC 3' (SEQ ID NO: 21)
P18: 5' ATCAGGCTGAAAATCTTCTCTCATCCGCCAAAACCTATCGACGCTTCCCCGCGC3' (SEQ ID NO: 22)

Method for Construction: Using YPG-002 or YPG-001, respectively, as a template, PCR amplification is performed with primers P17/P18 to provide a DNA fragment in 2017 bp comprising BBD29_04920 or BBD29_BBD29_04920^{C1560A} gene and the promoter thereof (as set forth in SEQ ID NO: 39 or SEQ ID NO: 40 for the sequence). Electrophoresis is performed on the amplified products for recovering a desired DNA fragment in 1947 bp using a Column DNA Gel Recovery Kit, which is ligated, using NEBuider recombination system, to shuttle plasmid pXMJ19 having been cleaved with *EcoR* I and recovered to provide an overexpression plasmid pXMJ19-BBD29_04920 or pXMJ19-BBD29_04920^{C1560A} . The plasmid contains a chloramphenicol-resistant marker, and the transformation of the plasmid into the strain can be achieved by screening with chloramphenicol.

PCR System: 10×Ex Taq Buffer 5 µL, dNTP Mixture (2.5 mM each) 4 µL, Mg²⁺ (25mM) 4 µL, primers (10pM) 2 µL each, Ex Taq (5U/µL) 0.25 µL, a template 1µL, and water as balance in a total volume of 50 µL.

The PCR amplification is performed by pre-denaturation for 5min at 94°C, denaturation for 30s at 94°C, annealing for 30s at 52°C, and extension for 90s at 72°C (30 circles), and overextension for 10min at 72°C.

The overexpression plasmid pXMJ19-BBD29_04920 is a recombinant vector obtained by inserting a DNA fragment (as set forth in SEQ ID NO: 39 for the sequence) containing a BBD29_04920 gene and a promoter thereof into the EcoR I recognition sites of the shuttle plasmid pXMJ19.

The overexpression plasmid pXMJ19-BBD29_04920^{C1560A} is a recombinant vector obtained by inserting a DNA fragment (as set forth in SEQ ID NO: 40 for the sequence) containing a BBD29_0492O^{C1560A} gene and a promoter thereof into the EcoR I recognition sites of the shuttle plasmid pXMJ19.

The two plasmids are each introduced into the strain *Corynebacterium glutamicum* CGMCC No. 21220 by virtue of electrotransformation, and PCR identification is performed on the cultured mono-colonies with primers M13R (-48) (5' AGCGGATAAC AATTTCACAC AGGA 3') and P18 for a transformed strain containing a fragment in about 2056 bp (as set forth in SEQ ID NO: 41 for the sequence without point mutation; as set forth in SEQ ID NO: 41 for the sequence with point mutation, except for position 1794 being A) from PCR amplification, designated as YPG-004 (without mutation point) and YPG-005 (with mutation point).

Recombinant bacterium YPG-004 contains a double-copy BBD29_04920 gene as set forth in SEQ ID NO: 1; recombinant bacterium YPG-004 is an engineered bacterium overexpressing a wild-type BBD29_04920 gene on a plasmid, i.e., overexpression outside a chromosome via plasmid pXMJ19-BBD29_04920.

Recombinant bacterium YPG-005 contains a mutated BBD29_0492O^{C1560A} gene as set forth in SEQ ID NO: 2; recombinant bacterium YPG-005 is an engineered bacterium overexpressing a mutant BBD29_04920^{C1560A} gene on a plasmid, i.e., overexpression outside a chromosome via plasmid pXMJ19-BBD29_04920^{C1560A}.

### Example 5. Construction of Engineered Strain with BBD29_04920 Gene Deleted on Genome

According to the genome sequence of *Corynebacterium glutamicum* ATCC13869 published on NCBI, two pairs of primers for amplifying fragments at both ends of a BBD29_04920 gene coding region are synthesized, as upstream and downstream homologous arm fragments. The primers are designed as follows (synthesized by Invitrogen Corporation, Shanghai):
P19: 5' CAGTGCCAAGCTTGCATGCCTGCAGGTCGACTCTAGTAAGGGGCAG TTGGTCTCG 3' (SEQ ID NO: 23)
P20: 5' GTATCAGGGGTTAAAAATTGCTTAATTTTCC CTGGCAGAA 3' (SEQ ID NO: 24)
P21: 5' TTCTGCCAGGGAAAATTAAGCAATTTTTAACCCCTGATAC 3' (SEQ ID NO: 25)

P22: 5' CAGCTATGACCATGATTACGAATTCGAGCTCGGTACCCATATCGCGCG ACATTGCGCG
3' (SEQ ID NO: 26)

Method for Construction: Using *Corynebacterium glutamicum* ATCC13869 as a template, PCR amplification is performed with primers P19/P20 and P21/P22, respectively, to provide an upstream homologous arm fragment in 733 bp (as set forth in SEQ ID NO: 42 for the sequence) and a downstream homologous arm fragment in 814 bp (as set forth in SEQ ID NO: 43 for the sequence). Further, P19/P22 are used as primers for an overlap PCR to provide an integral homologous arm fragment in 1507 bp (as set forth in SEQ ID NO: 44 for the sequence). After the PCR reaction is completed, electrophoresis is performed on the amplified products for recovering a desired DNA fragment in 1507 bp using a Column DNA Gel Recovery Kit, which is ligated, by virtue of NEBuider recombination system, to a shuttle plasmid, plasmid pk18mobsacB, having been cleaved with *Xba* I and recovered to provide a knockout plasmid. The plasmid contains a kanamycin-resistant marker.

The knockout plasmid is electrotransformed into the strain *Corynebacterium glutamicum* CGMCC No. 21220, and PCR identification is performed on the cultured mono-colonies each with the following primers (synthesized by Invitrogen Corporation, Shanghai):
P23: 5' TAAGGGGCAG TTGGTCTCG 3' (SEQ ID NO: 27)
P24: 5' ATATCGCGCG ACATTGCGCG 3' (SEQ ID NO: 28)

A strain with bands in 1433bp (as set forth in SEQ ID NO: 45 for the sequence) and 3380bp (as set forth in SEQ ID NO: 46 for the sequence) amplified from the above PCR amplification is a positive one, while a strain with just a band in 3380bp amplified therefrom is an original one. Having being screened on a culture medium with 15% sucrose, the positive strain is cultured on culture media with and without kanamycin, respectively, and strains that grow on a culture medium without kanamycin and do not grow on a culture medium with kanamycin are further subjected to PCR identification with primers P23/P24. The strain with a band in 1433 bp amplified therefrom is a genetically engineered strain with the BBD29_04920 gene coding region knocked out, designated as YPG-006.

Recombinant bacterium YPG-006 is a strain obtained by knocking out the BBD29_04920 gene coding region on the genome of *Corynebacterium glutamicum* CGMCC No. 21220.

### Example 6. Experiments on Fermentation of L-glutamic acid

Experiments on fermentation are performed on the strains YPG-001, YPG-002, YPG-003, YPG-004, YPG-005 and YPG-006 constructed in the Examples and an original strain *Corynebacterium glutamicum* CGMCC No. 21220 in a fermentation tank, BLBIO-5GC-4-H type (purchased from Shanghai Bailun Biological Technology Co., Ltd.) with the culturing medium (which is provided by dissolving the solutes shown in Table 1 in water) shown in Table 1 and the control process shown in Table 2, and fermentation products are collected.

At the initial moment when seeding is completed, the bacterium is at a concentration of 15 g/L in the system.

During the fermentation: sugar content in the system (residual sugar) is controlled by supplementing an aqueous solution containing 50-55% glucose.

Triplicates are made for each strain. Results are shown in Table 3.

**Table 1 Formulation of Culture Medium for Fermentation**

| Name of Reagent | Compounding Ratio |
|---|---|
| Glucose | 5.0 g/L |
| Phosphoric Acid | 0.38 g/L |
| Magnesium Sulfate | 1.85 g/L |
| Potassium Chloride | 1.6 g/L |
| Biotin | 550 µg/L |
| Vitamin B1 | 300 µg/L |
| Ferrous Sulfate | 10 mg/L |
| Manganese Sulfate | 10 g/dl |
| KH₂PO₄ | 2.8 g/L |
| Vitamin C | 0.75 mg/L |
| Vitamin B12 | 2.5 µg/L |
| Para-Aminobenzoic acid | 0.75 mg/L |
| Defoamer | 0.0015 ml/dl |
| Betaine | 1.5 g/L |
| Cane-Sugar Molasses | 7 ml/L |
| Corn Steep Liquor | 77 ml/L |
| Aspartic acid | 1.7 g/L |
| Hair powder | 2 g/L |

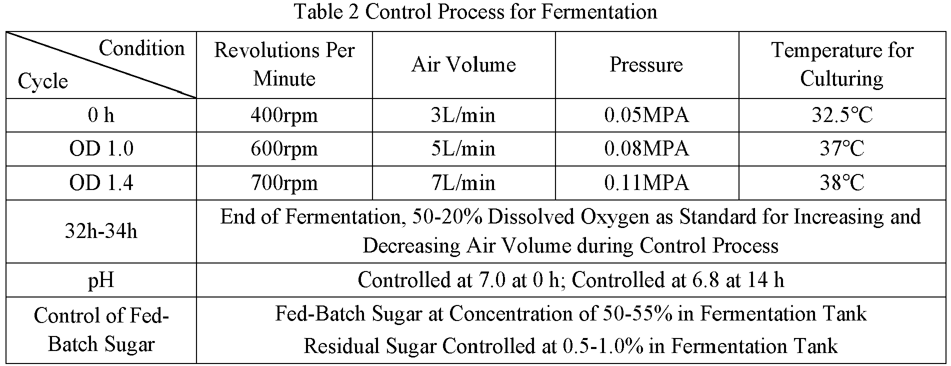

**Table 3 Results of Experiments on Fermentation of L-glutamic Acid**

| Strain | Production of L-Glutamic Acid (g/L) | OD (562nm) |
|---|---|---|
| *Corynebacterium glutamicum* CGMCC No. 21220 | 101.0 | 45.8 |
| YPG-001 | 106.5 | 44.3 |
| YPG-002 | 106.9 | 45.6 |
| YPG-003 | 106.2 | 46.4 |
| YPG-004 | 106.6 | 45.7 |
| YPG-005 | 105.8 | 45.5 |
| YPG-006 | 97.5 | 46.8 |

Results from Table 3 show that overexpression of the BBD29_04920 gene, or point mutation to the BBD29_04920 gene coding region, BBD29_04920^{C1560A}, and/or overexpression in *Corynebacterium glutamicum* facilitate an increase in the production of L-glutamic acid, while weakening or knocking out the gene is adverse to the accumulation of L-glutamic acid.

The Embodiments of the present invention are illustrated above. However, the present invention is not limited to the aforementioned Embodiments. Any modifications, equivalent replacements, improvements, etc. made within the spirit and principles of the present invention shall be encompassed within the scope of protection of the present invention.

### Industrial Application

The present invention has found out that, by weakening or knocking out the BBD29_04920 gene, the product coded by the gene has an effect on L-glutamic acid production capacity, and that a recombinant strain obtained by introducing a point mutation to the coding sequence, or by increasing the copy number of, or overexpressing the gene facilitates production of glutamic acid at a higher concentration as compared with an unmodified strain.

Specifically, the present invention first constructs a genetically engineered bacterium YPG-001with a point mutation (C-A) by introducing a point mutation to a BBD29_04920 gene coding region (SEQ ID NO: 1) of *Corynebacterium glutamicum* CGMCC No. 21220 via allelic replacement. In order to further investigate and verify that overexpressing a wild-type BBD29_04920 gene or a mutant gene BBD29_04920^{C1560A} thereof in a producer bacterium can increase the production of L-glutamic acid, an exogenous gene is integrated into the chromosome of a host, or expressed outside the chromosome by a plasmid, respectively, thereby constructing engineered bacteria YPG-002, YPG-003, YPG-004 and YPG-005 overexpressing BBD29_04920 gene or BBD29_04920^{C1560A} gene on genome and plasmid. Experiments suggest that BBD29_04920 gene and variants thereof are involved in the biosynthesis of L-glutamic acid. By overexpressing or knocking out BBD29_04920 gene, or having site-directed mutation (such as point mutation) thereto, the amount of accumulation of L-glutamic acid in a microorganism can be regulated. Point mutation to a BBD29_04920 gene coding region or overexpression of BBD29_04920 gene or a mutant gene BBD29_04920^{C1560A}thereof in a producer bacterium facilitates an increase in the production and conversion rate of L-glutamic acid, while knocking out or weakening the BBD29_04920 gene is adverse to the accumulation of L-glutamic acid. BBD29_04920 gene and a variant thereof (such as BBD29_04920^{C1560A} gene) can be used to construct a genetically engineered strain for producing L-glutamic acid to promote an increase in the production of L-glutamic acid, and to breed a high-production and high-quality strain for industrialized production, which finds values in a wide range of applications to, and is of important economic significance for industrialized production of L-glutamic acid.

## Claims

1. A bacterium for generating L-glutamic acid, **characterized in** having an improved expression of a polynucleotide encoding an amino acid sequence of SEQ ID NO: 3 or a homologous sequence thereof;
preferably, the improved expression is an enhanced expression of the polynucleotide encoding an amino acid sequence of SEQ ID NO: 3 or a homologous sequence thereof, or having a point mutation in the polynucleotide encoding an amino acid sequence of SEQ ID NO: 3 or a homologous sequence thereof, or having a point mutation in, and an enhanced expression of the polynucleotide encoding an amino acid sequence of SEQ ID NO: 3 or a homologous sequence thereof.

2. The bacterium of claim 1, **characterized in** a point mutation to the polynucleotide encoding an amino acid sequence of SEQ ID NO: 3 such that asparagine at position 520 in the amino acid sequence of SEQ ID NO: 3 is substituted with a different amino acid; preferably, asparagine at position 520 is substituted with lysine.

3. The bacterium of any one of claims 1-2, **characterized in that** the polynucleotide encoding an amino acid sequence of SEQ ID NO: 3 comprises a nucleotide sequence of SEQ ID NO: 1.

4. The bacterium of any one of claims 1-3, **characterized in that** the polynucleotide sequence having a point mutation is formed from a mutation to the base at position 1560 of a polynucleotide sequence set forth in SEQ ID NO: 1;
preferably, the mutation comprises a mutation of the base at position 1560 of a polynucleotide sequence set forth in SEQ ID NO: 1 from cytosine (C) to adenine (A);
preferably, the polynucleotide sequence having a point mutation comprises a polynucleotide sequence set forth in SEQ ID NO: 2.

5. The bacterium of any one of claims 1-4, **characterized in that** the bacterium is a bacterium of the genus *Corynebacterium,* preferably, *Corynebacterium acetoacidophilum, Corynebacterium acetoglutamicum, Corynebacterium callunae, Corynebacterium glutamicum, Brevibacterium flavum, Brevibacterium lactofermentum, Corynebacterium ammoniagenes, Corynebacterium pekinense, Brevibacterium saccharolyticum, Brevibacterium roseum,* and *Brevibacterium thiogenitalis;* more preferably, *Corynebacterium glutamicum* CGMCC No. 21220 or ATCC13869.

6. A polynucleotide sequence, **characterized in** comprising a polynucleotide encoding containing an amino acid sequence set forth in SEQ ID NO: 3, wherein asparagine at position 520 is substituted with a different amino acid; preferably, asparagine at position 520 is substituted with lysine;
preferably, the polynucleotide sequence comprises a polynucleotide encoding containing an amino acid sequence set forth in SEQ ID NO: 4;
preferably, the polynucleotide sequence is formed from a mutation to the base at position 1560 of a polynucleotide sequence set forth in SEQ ID NO: 1; preferably, the mutation is a mutation of the base at position 1560 of the polynucleotide sequence set forth in SEQ ID NO: 1 from cytosine (C) to adenine (A);
preferably, the polynucleotide sequence comprises a polynucleotide sequence set forth in SEQ ID NO: 2.

7. An amino acid sequence, **characterized in that** the sequence is set forth in SEQ ID NO: 4.

8. A recombinant vector, **characterized in** comprising the polynucleotide sequence of claim 6.

9. A recombinant strain, **characterized in** comprising the polynucleotide sequence of claim 6.

10. A method for producing L-glutamic acid, the method comprising: culturing the bacterium of any one of claims 1-5 and recovering L-glutamic acid from the culture.

11. A protein, **characterized in that** the protein is any one of:
A1) a protein whose amino acid sequence is SEQ ID NO: 4;
A2) a protein having 80% or more identity to and the same function as the protein indicated in A1), as obtained by subjecting the amino acid sequence set forth in SEQ ID NO: 4 to substitution and/or deletion and/or addition of amino acid residues;
A3) a fusion protein having the same function, as obtained by linking a tag to the N-terminus and/or C-terminus of A1) or A2).

12. A nucleic acid molecule, **characterized in that** the nucleic acid molecule is any one of:
B1) a nucleic acid molecule encoding the protein of claim 11;
B2) a DNA molecule whose coding sequence is set forth in SEQ ID NO: 2;
B3) a DNA molecule whose nucleotide sequence is set forth in SEQ ID NO: 2.

13. A biomaterial, **characterized in that** the biomaterial is any one of:
C1) an expression cassette comprising the nucleic acid molecule of claim 12;
C2) a recombinant vector comprising the nucleic acid molecule of claim 12, or a recombinant vector comprising the expression cassette of C1);
C3) a recombinant microorganism comprising the nucleic acid molecule of claim 12, or a recombinant microorganism comprising the expression cassette of C1), or a recombinant microorganism comprising the recombinant vector of C2).

14. Any one of the following uses of any one of D1)-D8):
F1) use of any one of D1)-D8) in the regulation of the production of L-glutamic acid of a microorganism;
F2) use of any one of D1)-D8) in the construction of a genetically engineered bacterium for producing L-glutamic acid;
F3) use of any one of D1)-D8) in the preparation of L-glutamic acid;
wherein the D1)-D8) is:
D1) the protein of claim 11;
D2) the nucleic acid molecule of claim 12;
D3) the biomaterial of claim 13;
D4) a DNA molecule whose nucleotide sequence is SEQ ID NO: 1;
D5) a DNA molecule having 90% or more identity to and the same function as the DNA molecule set forth in SEQ ID NO: 1, as obtained by subjecting the nucleotide sequence set forth in SEQ ID NO: 1 to modification, and/or substitution and/or deletion and/or addition of one or several nucleotides;
D6) an expression cassette comprising the DNA molecule in D4) or D5);
D7) a recombinant vector comprising the DNA molecule in D4) or D5), or a recombinant vector comprising the expression cassette of D6);
D8) a recombinant microorganism comprising the DNA molecule in D4) or D5), or a recombinant microorganism comprising the expression cassette of D6), or a recombinant microorganism comprising the recombinant vector of D7).

15. A method for increasing the production of L-glutamic acid in a microorganism, **characterized in that** the method comprises any one of:
E1) increasing the expression amount, or content of the nucleic acid molecule of claim 12 in a target microorganism to provide a microorganism having a greater production of L-glutamic acid than the target microorganism;
E2) increasing the expression amount, or content of the DNA molecule of D4) or D5) in claim 14 in a target microorganism to provide a microorganism having a greater production of L-glutamic acid than the target microorganism;
E3) performing a mutation on the DNA molecule whose nucleotide sequence is SEQ ID NO: 1 in the target microorganism to provide a microorganism having a greater production of L-glutamic acid than the target microorganism.

16. The method of claim 15, **characterized in that** the mutation is a point mutation.

17. The method of claim 16, **characterized in that** the point mutation is a mutation of methionine residue at position 199 in an amino acid sequence coded by the DNA molecule set forth in SEQ ID NO: 1 to another amino acid residue.

18. The method of claim 16 or 17, **characterized in that** the point mutation is a mutation of alanine at position 199 in an amino acid sequence coded by the DNA molecule set forth in SEQ ID NO: 1 to isoleucine, providing a mutated protein whose amino acid sequence is SEQ ID NO: 4.

19. A method for constructing the recombinant microorganism of claim 13 or 14, **characterized in that** the method comprises at least one of:
F1) introducing the nucleic acid molecule of claim 12 into a target microorganism to provide the recombinant microorganism;
F2) introducing the DNA molecule set forth in SEQ ID NO: 1 into a target microorganism to provide the recombinant microorganism;
F3) editing the DNA molecule set forth in SEQ ID NO: 1 with a gene editing measure to contain the DNA molecule set forth in SEQ ID NO: 2 in a target microorganism.

20. A method for preparing L-glutamic acid, **characterized in that** the method comprises producing L-glutamic acid with the recombinant microorganism of claim 13 or 14.
